# EUROPEAN PATENT SPECIFICATION

(11) **EP 3 795 089 B1**
(45) Date of publication and mention of the grant of the patent: **25.06.2025**
(21) Application number: 19804247.5
(22) Date of filing: 08.05.2019
(51) Int. Cl.: G06T 11/60, A61B 8/08, A61B 8/00, G16H 30/20, G16H 30/40, G16H 40/63

(54) **ULTRASOUND DIAGNOSIS DEVICE AND ULTRASOUND DIAGNOSIS DEVICE CONTROL METHOD**
ULTRASCHALLDIAGNOSEVORRICHTUNG UND VERFAHREN ZUR STEUERUNG DER ULTRASCHALLDIAGNOSEVORRICHTUNG
DISPOSITIF DE DIAGNOSTIC À ULTRASONS ET PROCÉDÉ DE COMMANDE DE DISPOSITIF DE DIAGNOSTIC À ULTRASONS

(30) Priority: 18.05.2018 JP 2018096253
(43) Date of publication of application: 24.03.2021
(73) Proprietor: FUJIFILM Corporation, Tokyo 106-8620 (JP)
(72) Inventor: TASHIRO Rika, kami-gun, Kanagawa 258-8538 (JP)
(74) Representative: Dehns
(86) International application number: PCT/JP2019/018385
(87) International publication number: WO 2019/220977

(56) References cited:
- JP-A- 2005 137 747
- JP-A- 2013 073 365
- JP-A- 2017 018 276
- JP-A- 2018 110 751
- US-A1- 2010 179 427
- US-A1- 2015 164 474

## Description

### BACKGROUND OF THE INVENTION

### 1. Field of the Invention

The present invention relates to an ultrasound diagnostic apparatus and a method for controlling the ultrasound diagnostic apparatus, and more particularly, to an ultrasound diagnostic apparatus comprising a touch sensor and a method for controlling the ultrasound diagnostic apparatus.

### 2. Description of the Related Art

In the related art, an ultrasound diagnostic apparatus using an ultrasound image has been put to practical use in the medical field. In general, this type of ultrasound diagnostic apparatus includes an ultrasound probe having a transducer array provided therein and an apparatus main body connected to the ultrasound probe. Ultrasonic waves are transmitted from the ultrasound probe to a subject. The ultrasound probe receives ultrasound echoes from the subject. The apparatus main body electrically processes a reception signal to generate an ultrasound image.

In recent years, for example, as disclosed in JP2015-198810A, an ultrasound diagnostic apparatus has come into widespread use in which an operation unit used by a user to perform an input operation comprises a touch sensor. In general, the touch sensor is disposed so as to be superimposed on a display screen of a display unit and is used for an input operation by a so-called touch operation of bringing a user's finger, a stylus pen, and the like into contact with or close to the display screen. According to the ultrasound diagnostic apparatus disclosed in JP2015-198810A, a caliper that is displayed so as to be superimposed on the ultrasound diagnostic image on the display unit can be moved by a touch operation to measure, for example, the distance on the ultrasound image. US 2010/179427 is another document that discloses an ultrasonic diagnostic apparatus comprising a touch panel.

### SUMMARY OF THE INVENTION

However, in the ultrasound diagnostic apparatus disclosed in JP2015-198810A, in a case in which the touch operation is performed on the caliper, for example, there is a concern that the caliper displayed so as to be superimposed on the ultrasound diagnostic image will not be touched, but the ultrasound diagnostic image displayed below the caliper will be touched due to, for example, the slight deviation of the touch position of the user's finger, the stylus pen, or the like from the display position of the caliper. As such, in a case in which the input operation that is not intended by the user is performed, the convenience of the touch operation is reduced.

The invention has been made in order to solve the problems of the related art, and an object of the invention is to provide an ultrasound diagnostic apparatus and a method for controlling the ultrasound diagnostic apparatus that can improve convenience in a case in which a user performs a touch operation.

In order to achieve the object, according to the invention, there is provided an ultrasound diagnostic apparatus as claimed in claim 1.

Preferably, the movable operation target is a region of interest for designating a partial region of the ultrasound diagnostic image in a case in which an inspection is performed in a brightness mode or a color Doppler mode.

Alternatively, the movable operation target may be a cursor for designating a position where a motion mode image is acquired in the ultrasound diagnostic image displayed in a brightness mode in a case in which an inspection is performed in a motion mode.

Alternatively, the movable operation target may be a cursor and a gate for designating a position where a pulse Doppler mode image is acquired in the ultrasound diagnostic image displayed in a brightness mode in a case in which an inspection is performed in a pulse Doppler mode.

Alternatively, the movable operation target may be an annotation.

Alternatively, the movable operation target may be a body mark and a probe mark that is displayed on the body mark.

Alternatively, the movable operation target may be a caliper for measurement.

Further, the ultrasound diagnostic apparatus may further comprise an ultrasound probe and a diagnostic apparatus main body that are wirelessly connected to each other. The ultrasound probe may include: a transducer array; a transmitting and receiving unit that transmits ultrasonic waves from the transducer array and generates a sound ray signal on the basis of a reception signal acquired by the transducer array; an image information data generation unit that generates image information data on the basis of the sound ray signal generated by the transmitting and receiving unit; and a wireless communication unit that wirelessly transmits the image information data generated by the image information data generation unit to the diagnostic apparatus main body. The diagnostic apparatus main body may include: the display unit that displays an ultrasound image on the basis of the image information data wirelessly transmitted from the ultrasound probe; the operation unit; the operation target generation unit; and the operation target receiving unit.

In this case, preferably, the image information data is a signal obtained by performing attenuation correction according to a depth of a reflection position of the ultrasonic waves and an envelope detection process on the sound ray signal generated by the transmitting and receiving unit.

Alternatively, preferably, the image information data is an ultrasound image signal obtained by performing attenuation correction according to a depth of a reflection position of the ultrasonic waves and an envelope detection process on the sound ray signal generated by the transmitting and receiving unit and converting the sound ray signal according to a predetermined image display method.

According to the invention, there is provided a method for controlling an ultrasound diagnostic apparatus as claimed in claim 11.

According to the invention, the ultrasound diagnostic apparatus comprises the operation target generation unit that generates an operation target displayed on the display unit according to an operation by the operation unit; and the operation target receiving unit that, in a case in which the operation target generation unit generates an operation target that is superimposed on the ultrasound diagnostic image displayed in the image display region and is movable, receives an operation performed on any position in the image display region through the operation unit as an operation of selecting the movable operation target. Therefore, it is possible to improve convenience in a case in which the user performs a touch operation.

### BRIEF DESCRIPTION OF THE DRAWINGS

Fig. 1 is a block diagram illustrating a configuration of an ultrasound diagnostic apparatus according to Embodiment 1 of the invention.
Fig. 2 is a block diagram illustrating an internal configuration of a receiving unit in Embodiment 1 of the invention.
Fig. 3 is a diagram schematically illustrating an image display region set in a display unit in Embodiment 1 of the invention.
Fig. 4 is a diagram illustrating an example of movable operation targets displayed in the image display region of the display unit in Embodiment 1 of the invention.
Fig. 5 is a diagram schematically illustrating an example of a plurality of movable operation targets displayed in an image display region of a display unit in Embodiment 2 of the invention.
Fig. 6 is a block diagram illustrating a configuration of an ultrasound diagnostic apparatus according to Embodiment 3 of the invention.

### DESCRIPTION OF THE PREFERRED EMBODIMENTS

Embodiments of the invention will be described below with reference to the accompanying drawings.

### Embodiment 1

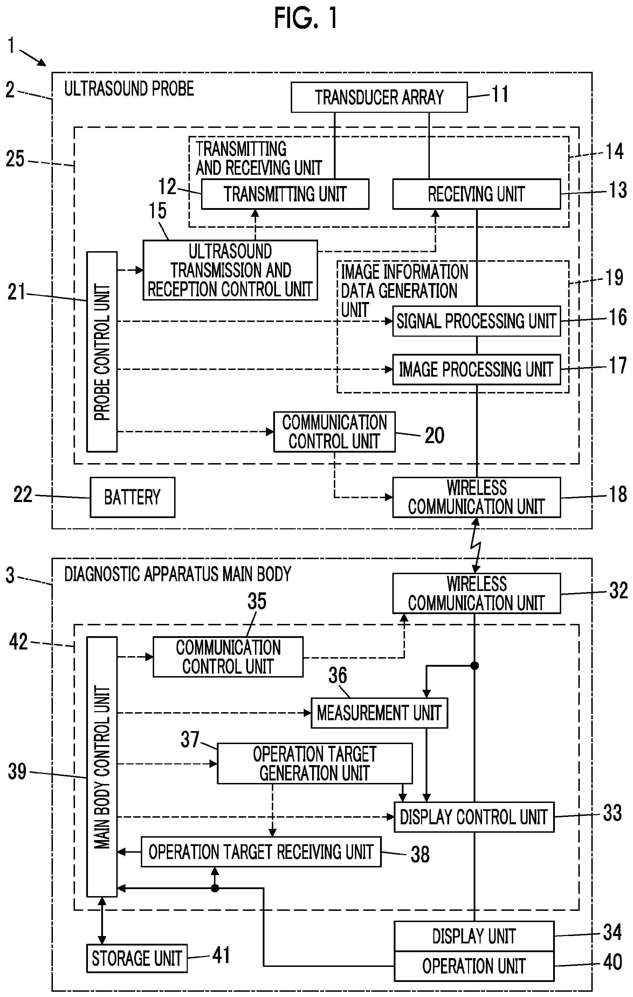

Fig. 1 illustrates the configuration of an ultrasound diagnostic apparatus 1 according to Embodiment 1 of the invention. As illustrated in Fig. 1, the ultrasound diagnostic apparatus 1 comprises an ultrasound probe 2 and a diagnostic apparatus main body 3. The ultrasound probe 2 and the diagnostic apparatus main body 3 are connected by wireless communication.

The ultrasound probe 2 of the ultrasound diagnostic apparatus 1 comprises a transducer array 11. The transducer array 11 is connected to a transmitting unit 12 and a receiving unit 13. The transmitting unit 12 and the receiving unit 13 form a transmitting and receiving unit 14. An ultrasound transmission and reception control unit 15 is connected to the transmitting unit 12 and the receiving unit 13. A signal processing unit 16, an image processing unit 17, and a wireless communication unit 18 are sequentially connected to the receiving unit 13. The signal processing unit 16 and the image processing unit 17 form an image information data generation unit 19.

Further, a communication control unit 20 is connected to the wireless communication unit 18. A probe control unit 21 is connected to the ultrasound transmission and reception control unit 15, the signal processing unit 16, the image processing unit 17, and the communication control unit 20. Further, the ultrasound probe 2 has a battery 22 provided therein. A probe-side processor 25 is configured by the transmitting and receiving unit 14, the ultrasound transmission and reception control unit 15, the image information data generation unit 19, the communication control unit 20, and the probe control unit 21.

The diagnostic apparatus main body 3 of the ultrasound diagnostic apparatus 1 comprises a wireless communication unit 32. A display control unit 33 and a display unit 34 are sequentially connected to the wireless communication unit 32. Further, a communication control unit 35 and a measurement unit 36 are connected to the wireless communication unit 32, and the measurement unit 36 is connected to the display control unit 33. An operation target generation unit 37 is connected to the display control unit 33. Further, an operation unit 40 is disposed so as to be superimposed on the display unit 34, and an operation target receiving unit 38 is connected to the operation unit 40. The operation target receiving unit 38 is connected to the operation target generation unit 37.

A main body control unit 39 is connected to the display control unit 33, the communication control unit 35, the measurement unit 36, the operation target generation unit 37, the operation target receiving unit 38, and the operation unit 40. A storage unit 41 is connected to the main body control unit 39 such that information can be bidirectionally transmitted and received.

Further, a main-body-side processor 42 is configured by the display control unit 33, the communication control unit 35, the measurement unit 36, the operation target generation unit 37, the operation target receiving unit 38, and the main body control unit 39.

The transducer array 11 of the ultrasound probe 2 has a plurality of ultrasound transducers which are arranged one-dimensionally or two-dimensionally. Each of the transducers transmits ultrasonic waves according to a driving voltage signal supplied from the transmitting unit 12, receives waves reflected from a subject, and outputs a reception signal. Each transducer is configured using an element in which electrodes are formed at both ends of a piezoelectric body consisting of, for example, a piezoelectric ceramic typified by lead zirconate titanate (PZT), a polymeric piezoelectric element typified by polyvinylidene difluoride (PVDF), and a piezoelectric single crystal typified by lead magnesium niobate-lead titanate (PMN-PT).

The transmitting unit 12 of the transmitting and receiving unit 14 includes, for example, a plurality of pulse generators, adjusts the amount of delay of each driving signal on the basis of a transmission delay pattern selected according to a control signal from the ultrasound transmission and reception control unit 15 such that the ultrasonic waves transmitted from the plurality of transducers of the transducer array 11 form an ultrasound beam, and supplies the driving signals to the plurality of transducers. As such, in a case in which a pulsed or continuous-wave voltage is applied to the electrodes of the transducers of the transducer array 11, the piezoelectric body is expanded and contracted and pulsed or continuous ultrasonic waves are generated from each transducer. An ultrasound beam is formed from a combined wave of the ultrasonic waves.

The transmitted ultrasound beam is reflected by a target, such as a part of the subject, and is propagated toward the transducer array 11. The ultrasonic waves propagated toward the transducer array 11 in this way are received by each of the ultrasound transducers forming the transducer array 11. In this case, each of the ultrasound transducers forming the transducer array 11 receives propagated ultrasound echoes, is expanded and contracted to generate an electric signal, and outputs a reception signal which is the electric signal to the receiving unit 13.

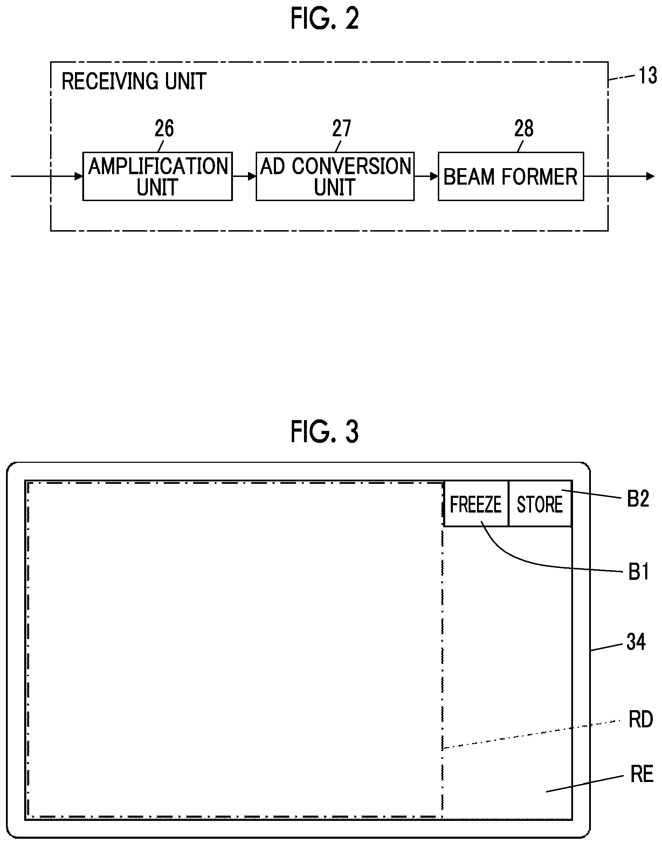

The receiving unit 13 of the transmitting and receiving unit 14 processes the reception signal output from the transducer array 11 according to a control signal from the ultrasound transmission and reception control unit 15. As illustrated in Fig. 2, the receiving unit 13 has a configuration in which an amplification unit 26, an analog digital (AD) conversion unit 27, and a beam former 28 are connected in series to each other. The amplification unit 26 amplifies the reception signal input from each of the transducers forming the transducer array 11 and transmits the amplified reception signal to the AD conversion unit 27. The AD conversion unit 27 converts the reception signal transmitted from the amplification unit 26 into digital element data and transmits the element data to the beam former 28. The beam former 28 performs a reception focusing process which gives a delay to each element data item following the set sound velocity on the basis of a reception delay pattern selected according to a control signal from the ultrasound transmission and reception control unit 15 and performs addition (phasing addition). The sound ray signal in which the focus of the ultrasound echo is narrowed is generated by the reception focusing process.

The ultrasound transmission and reception control unit 15 of the probe-side processor 25 controls the transmitting unit 12 and the receiving unit 13 of the transmitting and receiving unit 14 to perform the transmission of ultrasound beams and the reception of ultrasound echoes on the basis of an inspection mode and a scanning method instructed by the probe control unit 21. Here, it is assumed that the inspection mode indicates any one of the inspection modes that can be used in the ultrasound diagnostic apparatus, such as a brightness (B) mode, a motion (M) mode, a color Doppler (CD) mode, a power Doppler (PD) mode, a pulse Doppler (PW) mode, and a continuous wave Doppler (CW), and the scanning method indicates any one of scanning methods, such as an electronic sector scanning method, an electronic linear scanning method, and an electronic convex scanning method.

The signal processing unit 16 of the image information data generation unit 19 corrects the attenuation of the sound ray signal generated by the beam former 28 of the receiving unit 13 caused by a propagation distance according to the depth of the position where the ultrasonic waves are reflected and performs an envelope detection process on the sound ray signal to generate a signal which is tomographic image information related to the tissues in the subject.

The image processing unit 17 of the image information data generation unit 19 raster-converts the signal generated by the signal processing unit 16 into an image signal following a general television signal scanning method, performs various types of necessary image processing, such as brightness correction, gradation correction, sharpness correction, and color correction, on the generated image signal to generate an ultrasound image signal, and transmits the ultrasound image signal as image information data to the wireless communication unit 18 of the ultrasound probe 2.

The wireless communication unit 18 of the ultrasound probe 2 is configured by, for example, a circuit including an antenna for transmitting and receiving radio waves and performs wireless communication with the wireless communication unit 32 of the diagnostic apparatus main body 3. In this case, the wireless communication unit 18 of the ultrasound probe 2 modulates a carrier on the basis of the ultrasound image signal generated by the image processing unit 17 of the image information data generation unit 19 to generate a transmission signal indicating the ultrasound image signal and wirelessly transmits the generated transmission signal to the wireless communication unit 32 of the diagnostic apparatus main body 3. For example, amplitude shift keying (ASK), phase shift keying (PSK), quadrature phase shift keying (QPSK), and 16 quadrature amplitude modulation (16QAM) are used as the carrier modulation method.

The probe control unit 21 of the probe-side processor 25 controls each unit of the ultrasound probe 2 on the basis of, for example, a program stored in advance.

The battery 22 of the ultrasound probe 2 is provided in the ultrasound probe 2 and supplies power to each circuit of the ultrasound probe 2.

The communication control unit 20 of the probe-side processor 25 controls the wireless communication unit 18 of the ultrasound probe 2 such that the ultrasound image signal is transmitted with transmission radio field intensity set by the probe control unit 21.

The wireless communication unit 32 of the diagnostic apparatus main body 3 is configured by, for example, a circuit including an antenna for transmitting and receiving radio waves and performs wireless communication with the wireless communication unit 18 of the ultrasound probe 2. In this case, the wireless communication unit 32 of the diagnostic apparatus main body 3 receives, for example, the transmission signal indicating the ultrasound image signal wirelessly transmitted from the wireless communication unit 18 of the ultrasound probe 2 through the antenna, demodulates the received transmission signal, and outputs the ultrasound image signal.

Further, the communication control unit 35 of the main-body-side processor 42 controls the wireless communication unit 32 of the diagnostic apparatus main body 3 such that the transmission signal is received from the wireless communication unit 18 of the ultrasound probe 2.

The display control unit 33 of the main-body-side processor 42 performs predetermined processing on the ultrasound image signal output from the wireless communication unit 32 of the diagnostic apparatus main body 3 and displays an ultrasound diagnostic image on the display unit 34 under the control of the main body control unit 39. In addition to the ultrasound diagnostic image, the display control unit 33 displays, for example, operation targets generated by the operation target generation unit 37 and measurement results calculated by the measurement unit 36 on the display unit 34, which will be described below.

The display unit 34 of the diagnostic apparatus main body 3 displays, for example, the ultrasound diagnostic image under the control of the display control unit 33. In this case, as illustrated in Fig. 3, an image display region RD for displaying the ultrasound diagnostic image is set in the display unit 34, and the ultrasound diagnostic image is displayed in the image display region RD. In the display unit 34, for example, buttons used for an input operation are displayed in an outer region RE that is set outside the image display region RD. In the example illustrated in Fig. 3, a freeze button B1 for the freeze display of the ultrasound diagnostic image and a storage button B2 for storing the ultrasound diagnostic image displayed in the image display region RD are displayed in the outer region RE.

The display unit 34 includes a display device such as a liquid crystal display (LCD) or an organic electroluminescence (EL) display.

The operation unit 40 of the diagnostic apparatus main body 3 is used by the user to perform an input operation and includes a touch sensor that is disposed so as to be superimposed on the display unit 34. Input information input by the user through the touch sensor of the operation unit 40 is transmitted to the operation target receiving unit 38 and the main body control unit 39.

The operation target generation unit 37 of the main-body-side processor 42 generates operation targets displayed on the display unit 34 according to an input operation of the user through the operation unit 40. Here, the operation targets are targets on which the user can perform an input operation through the operation unit 40. The operation targets include targets displayed in the image display region RD and targets displayed in the outer region RE such as the freeze button B1 and the storage button B2. For example, the image display region RD includes a target which is superimposed on the ultrasound diagnostic image and is movable, such as a caliper for measuring the distance on the ultrasound diagnostic image, and a target which is superimposed on the ultrasound diagnostic image and whose position is fixed, such as a button for displaying a menu in a case in which the inspection mode is selected.

In a case in which the operation target generation unit 37 generates the operation targets that are displayed so as to be superimposed on the ultrasound diagnostic image displayed in the image display region RD of the display unit 34 and is movable, the operation target receiving unit 38 of the main-body-side processor 42 receives an operation performed on any position in the image display region RD by the user through the operation unit 40 as an operation of selecting the movable operation target. Then, in the image display region RD, the operation of operation targets other than the operation target currently operated by the user through the operation unit 40 is prohibited. Therefore, only the input operation intended by the user is performed.

The measurement unit 36 of the main-body-side processor 42 measures, for example, the distance on the basis of the operation target which has been generated by the operation target generation unit 37 and operated by the user through the operation unit 40, such as a caliper for measuring the distance on the ultrasound diagnostic image. The measurement unit 36 transmits the calculated measurement result to the display control unit 33.

The main body control unit 39 of the main-body-side processor 42 controls each unit of the diagnostic apparatus main body 3 on the basis of the program stored in advance in, for example, the storage unit 41 and the input operation of the user through the operation unit 40.

The storage unit 41 of the diagnostic apparatus main body 3 stores, for example, an operation program for the diagnostic apparatus main body 3. The following can be used as the storage unit 41: a recording medium, such as a hard disc drive (HDD), a solid state drive (SSD), a flexible disc (FD), a magneto-optical disc (MO disc), a magnetic tape (MT), a random access memory (RAM), a compact disc (CD), a digital versatile disc (DVD), a secure digital card (SD card), or a universal serial bus memory (USB memory); or a sever.

Here, the probe-side processor 25 including the transmitting and receiving unit 14, the ultrasound transmission and reception control unit 15, the image information data generation unit 19, the communication control unit 20, and the probe control unit 21 in the ultrasound probe 2 and the main-body-side processor 42 including the display control unit 33, the communication control unit 35, the measurement unit 36, the operation target generation unit 37, the operation target receiving unit 38, and the main body control unit 39 in the diagnostic apparatus main body 3 are implemented by a central processing unit (CPU) and a control program for causing the CPU to perform various processes. However, the processors may be implemented by a field programmable gate array (FPGA), a digital signal processor (DSP), an application specific integrated circuit (ASIC), a graphics processing unit (GPU), other integrated circuits (ICs), or combinations thereof.

Some or all of the transmitting and receiving unit 14, the ultrasound transmission and reception control unit 15, the communication control unit 20, and the probe control unit 21 of the probe-side processor 25 may be integrated into, for example, one CPU. Similarly, some or all of the display control unit 33, the communication control unit 35, the measurement unit 36, the operation target generation unit 37, the operation target receiving unit 38, and the main body control unit 39 of the main-body-side processor 42 may be integrated into, for example, one CPU.

Next, the operation of the ultrasound diagnostic apparatus 1 according to Embodiment 1 of the invention will be described. Here, for the sake of description, the operation of the ultrasound diagnostic apparatus 1 in a case in which the distance between two points on the ultrasound diagnostic image is measured will be described below.

The ultrasound transmission and reception control unit 15 of the probe-side processor 25 controls the transmitting and receiving unit 14 such that the transducer array 11 transmits and receives ultrasonic waves on the basis of a predetermined inspection mode under the control of the probe control unit 21. Here, in the following description, for the sake of description, it is assumed that a B-mode is used as the predetermined inspection mode used for ultrasound diagnosis.

In this case, ultrasound beams are transmitted into the subject from the plurality of ultrasound transducers of the transducer array 11 according to a driving signal from the transmitting unit 12 of the transmitting and receiving unit 14 under the control of the ultrasound transmission and reception control unit 15. Ultrasound echoes from the subject, which are based on the transmitted ultrasound beams, are received by each ultrasound transducer and a reception signal which is an analog signal is output to the receiving unit 13, is amplified by the amplification unit 26, and is converted into a digital signal by the AD conversion unit 27. As a result, reception data is acquired. The beam former 28 performs a reception focusing process on the reception data to generate a sound ray signal corresponding to each frame of the ultrasound image.

The signal processing unit 16 of the image information data generation unit 19 performs attenuation correction and an envelope detection process on the sound ray signal generated by the beam former 28 of the receiving unit 13 to generate a signal which is tomographic image information related to the tissues in the subject. The image processing unit 17 of the image information data generation unit 19 performs raster conversion on the signal generated by the signal processing unit 16 and further performs various types of necessary image processing on the signal to generate an ultrasound image signal as image information data.

The ultrasound image signal generated in the image information data generation unit 19 is transmitted to the wireless communication unit 18 of the ultrasound probe 2 and is then wirelessly transmitted as a transmission signal from the wireless communication unit 18 of the ultrasound probe 2 to the wireless communication unit 32 of the diagnostic apparatus main body 3.

The transmission signal wirelessly transmitted from the wireless communication unit 18 of the ultrasound probe 2 is demodulated by the wireless communication unit 32 of the diagnostic apparatus main body 3 and is then transmitted as the ultrasound image signal to the display control unit 33 and the measurement unit 36 of the main-body-side processor 42.

The ultrasound image signal transmitted to the display control unit 33 is displayed as an ultrasound diagnostic image on the display unit 34 of the diagnostic apparatus main body 3 under the control of the display control unit 33. As illustrated in Fig. 3, in the display unit 34, the image display region RD for displaying the ultrasound diagnostic image and the outer region RE outside the image display region RD are set, and the ultrasound diagnostic image is displayed in the image display region RD.

For example, the ultrasound diagnostic image displayed in the image display region RD of the display unit 34 is enlarged, minified, or moved by a touch operation of the user through the operation unit 40 under the control of the display control unit 33 and the main body control unit 39. For example, in a case in which the user moves the ultrasound diagnostic image displayed on the display unit 34 while touching the ultrasound diagnostic image with a finger, a stylus pen or the like, the ultrasound diagnostic image displayed on the display unit 34 is also moved following the user's finger or the stylus pen.

Further, command information to display the operation target on the display unit 34 can be input by an input operation of the user through the operation unit 40 or according to a predetermined inspection sequence in a state in which the ultrasound diagnostic image is displayed in the image display region RD of the display unit 34. The command information to display the operation target is transmitted to the main body control unit 39. Under the control of the main body control unit 39, the operation target generation unit 37 generates an operation target corresponding to the input operation of the user through the operation unit 40 and the generated operation target is displayed on the display unit 34 through the display control unit 33.

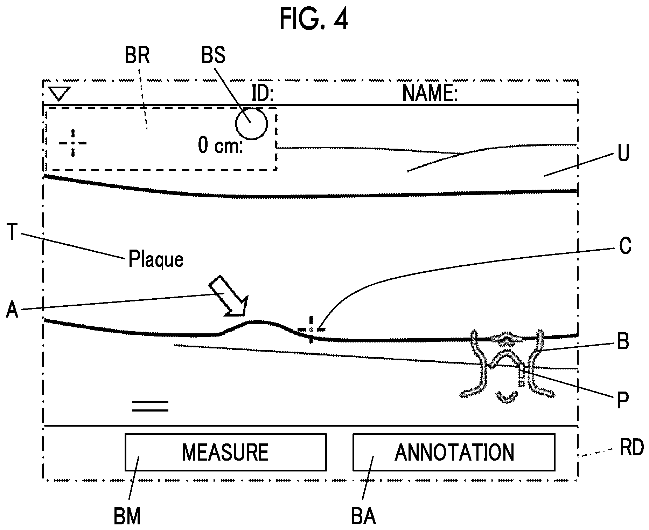

For example, as illustrated in Fig. 4, in a case in which the user touches a measurement button BM through the operation unit 40 to input command information to measure the distance between two points on an ultrasound diagnostic image U, the command information input by the user is transmitted to the main body control unit 39. Further, under the control of the main body control unit 39, the operation target generation unit 37 generates a first caliper C illustrated in Fig. 4 and a second caliper (not illustrated) as the operation targets. First, the first caliper C is displayed on the display unit 34 through the display control unit 33. In this case, the first caliper C is displayed so as to be superimposed on the ultrasound diagnostic image U displayed in the image display region RD of the display unit 34 and to be movable. The second caliper is disposed at the same display position as the first caliper C and is displayed in the image display region RD in a case in which the first caliper C is moved.

As such, in a case in which the operation target generation unit 37 generates the operation target that is superimposed on the ultrasound diagnostic image U displayed in the image display region RD of the display unit 34 and is movable, the operation target receiving unit 38 receives an operation performed on any position in the image display region RD by the user through the operation unit 40 as the operation of selecting the movable operation target.

For example, as illustrated in Fig. 4, in a case in which the first caliper C is displayed so as to be superimposed on the ultrasound diagnostic image U in the image display region RD of the display unit 34, the operation received by the operation target receiving unit 38 is not limited to the operation on the display position of the first caliper C, and the operation target receiving unit 38 receives a touch operation on any position in the image display region RD as the operation of selecting and moving the first caliper C. That is, the operation target receiving unit 38 prohibits, for example, the operations of enlarging, minifying, and moving the ultrasound diagnostic image U and receives only the operation on the first caliper C. Therefore, for example, even in a case in which the user moves a position on the ultrasound diagnostic image U that deviates from the display position of the first caliper C while touching the position with the finger, the stylus pen or the like, it is possible to perform an operation on the first caliper C without moving the ultrasound diagnostic image U or without ending the measurement operation.

In the invention, as such, the operation target receiving unit 38 prohibits the operation of operation targets other than the operation target intended by the user, and the operation is fixed to only the operation target intended by the user. Therefore, it is possible to improve convenience in a case in which the user performs the touch operation.

In a case in which the first caliper C is moved by the touch operation of the user and the user's finger, the stylus pen, or the like used to operate the first caliper C is taken off the image display region RD of the display unit 34, the position of the first caliper C is determined. In this state, the second caliper is displayed at the initial position of the first caliper C on the image display region RD. For example, the operation target receiving unit 38 receives an operation on any position in the image display region RD as the operation of selecting and moving the second caliper, using the tapping of the second caliper by the user as a trigger. For example, the operation may be fixed only to the second caliper, using the elapse of a predetermined time, such as two seconds, since the taking-off of the user's finger, the stylus pen, or the like used to operate the first caliper C from the image display region RD as a trigger. In addition, the position of the second caliper is determined in the same manner as the first caliper C.

In a case in which the positions of two calipers for measuring the distance between two points on the ultrasound diagnostic image U are determined, the measurement unit 36 of the main-body-side processor 42 measures the distance between the two calipers. The measurement result, such as a measurement value calculated by the measurement unit 36, is displayed on the display unit 34 through the display control unit 33. For example, as illustrated in Fig. 4, the measurement result is displayed in a measurement result box BR that is superimposed on the ultrasound diagnostic image U in the image display region RD of the display unit 34.

In this way, the operation of the ultrasound diagnostic apparatus 1 ends.

As described above, according to the ultrasound diagnostic apparatus 1 of Embodiment 1 of the invention, in a case in which the operation target generation unit 37 generates the operation target that is superimposed on the ultrasound diagnostic image U displayed in the image display region RD of the display unit 34 and is movable, the operation target receiving unit 38 receives an operation performed on any position in the image display region RD by the user through the operation unit 40 as the operation of selecting and moving the movable operation target. Therefore, it is possible to prevent an input operation that is not intended by the user and to improve convenience in a case in which a touch operation is performed.

In Embodiment 1, the caliper for measuring the distance between two points on the ultrasound diagnostic image U is given as an example of the operation target that is superimposed on the ultrasound diagnostic image U and is movable. However, the movable operation target is not limited thereto.

For example, the operation target generation unit 37 may generate a closed section, such as a circle or an ellipse, for measuring the area on the ultrasound diagnostic image U as the movable operation target and may display the closed section in the image display region RD of the display unit 34 through the display control unit 33. In a case in which the closed section is displayed in the image display region RD, the operation target receiving unit 38 can fix the operation of the user only to the operation of, for example, selecting and moving the closed section.

Further, for example, the measurement result box BR illustrated in Fig. 4 may be displayed as the movable operation target in the image display region RD. In this case, the operation target generation unit 37 can generate a measurement result movement button BS associated with the measurement result box BR and display the measurement result movement button BS in the image display region RD. The operation target receiving unit 38 can fix the operation of the user only to the operation of, for example, selecting and moving the measurement result box BR, using the touch of the user with the measurement result movement button BS as a trigger.

In addition, for example, the operation target generation unit 37 may generate annotations, such as a text T and an arrow A illustrated in Fig. 4, as the movable operation targets, and display the annotations in the image display region RD of the display unit 34 through the display control unit 33. For example, as illustrated in Fig. 4, an annotation button BA for displaying annotations is displayed in the image display region RD. The operation target generation unit 37 generates the text A of "Plaque" and the arrow A as the annotations, using the touch of the annotation button BA by the user as a trigger. In addition, in a case in which the annotations are displayed in the image display region RD, the operation target receiving unit 38 can fix the operation of the user to only the operation of, for example, selecting and moving the annotations.

Further, for example, the operation target generation unit 37 may generate a so-called body mark B and a probe mark P illustrated in Fig. 4 as the movable operation targets and display them in the image display region RD of the display unit 34 through the display control unit 33. For example, a button (not illustrated) for displaying the body mark B and the probe mark P is displayed in the image display region RD or the outer region RE of the display unit 34. The operation target generation unit 37 generates the body mark B and the probe mark P, using the touch of the button by the user as a trigger, as illustrated in Fig. 4. In a case in which the body mark B and the probe mark P are displayed in the image display region RD, the operation target receiving unit 38 can fix the operation of the user to only the operation of, for example, selecting and moving the body mark B and the probe mark P.

Here, as illustrated in Fig. 4, in a case in which a plurality of movable operation targets including the first caliper C, the measurement result box BR, the annotations, such as the text T and the arrow A, the body mark B, and the probe mark P are displayed in the image display region RD, for example, the operation target receiving unit 38 can fix the operation of the operation targets according to a predetermined sequence of fixing the operations in the order of the first caliper C, the measurement result box BR, the annotations, such as the text T and the arrow A, the body mark B, and the probe mark P. As such, in a case in which a plurality of movable operation targets are displayed in the image display region RD at the same time, the operation target receiving unit 38 can sequentially fix the operation of the movable operation targets according to, for example, a predetermined sequence. The predetermined sequence of fixing the operation targets is not limited to the sequence of fixing the operation targets in the order of the first caliper C, the measurement result box BR, the text T, the arrow A, the body mark B, and the probe mark P illustrated as an example here.

In addition, for example, in a case in which the B-mode and the CD-mode are used as the inspection mode used for ultrasound diagnosis, the operation target generation unit 37 can generate a region of interest for designating a partial region of the ultrasound diagnostic image U as the movable operation target and display the region of interest in the image display region RD of the display unit 34 through the display control unit 33. In a case in which the region of interest is displayed in the image display region RD, the operation target receiving unit 38 can fix the operation of the user to only the operation of, for example, selecting and moving the region of interest.

In a case in which the M-mode is used as the inspection mode used for ultrasound diagnosis, for example, a B-mode image and an M-mode image are displayed in the image display region RD of the display unit 34. For example, in this case, the operation target generation unit 37 can generate a cursor for designating a position where the M-mode image is acquired on the B-mode image as the movable operation target and display the cursor in the image display region RD through the display control unit 33. In a case in which the cursor for designating the position where the M-mode image is acquired on the B-mode image is displayed in the image display region RD, the operation target receiving unit 38 can fix the operation of the user to only the operation of, for example, selecting and moving the cursor.

In a case in which the PW-mode is used as the inspection mode used for ultrasound diagnosis, for example, a B-mode image and a PW-mode image are displayed in the image display region RD of the display unit 34. For example, in this case, the operation target generation unit 37 can generate a cursor for designating a position where the PW-mode image is acquired on the B-mode image as the movable operation target and display the cursor in the image display region RD through the display control unit 33. In a case in which the cursor for designating the position where the PW-mode image is acquired on the B-mode image is displayed in the image display region RD, the operation target receiving unit 38 can fix the operation of the user to only the operation of, for example, selecting and moving the cursor.

Further, in a case in which the PW-mode is used as the inspection mode used for ultrasound diagnosis, for example, the operation target generation unit 37 can generate, as the movable operation target, a so-called Doppler gate for performing measurement related to a blood flow which is displayed on the B-mode image. For example, a button for measuring a blood flow rate, a blood flow volume, and the like is displayed in the image display region RD or the outer region RE of the display unit 34, which is not illustrated. The operation target generation unit 37 generates the Doppler gate, using the touch of the button by the user as a trigger. Further, in a case in which the Doppler gate is displayed in the image display region RD, the operation target receiving unit 38 can fix the operation of the user only to operation of, for example, selecting and moving the Doppler gate.

Furthermore, in Embodiment 1, the ultrasound diagnostic image U is displayed on the display unit 34 of the diagnostic apparatus main body 3 on the basis of the image information data generated by the ultrasound probe 2, and the movable operation targets are displayed so as to be superimposed on the ultrasound diagnostic image U. However, the ultrasound diagnostic image stored in advance may be displayed on the display unit 34. For example, the diagnostic apparatus main body 3 may be provided with an image memory for storing ultrasound diagnostic images, which is not illustrated. The ultrasound diagnostic image stored in the image memory may be displayed on the display unit 34 and the movable operation target may be displayed so as to be superimposed on the ultrasound diagnostic image.

### Embodiment 2

In Embodiment 1, in a case in which the movable operation target is displayed in the image display region RD of the display unit 34, the operation target receiving unit 38 receives the operation of the displayed operation target. However, in a case in which a plurality of operation targets are displayed in the image display region RD, the operation targets whose operation is received by the operation target receiving unit 38 may be switched.

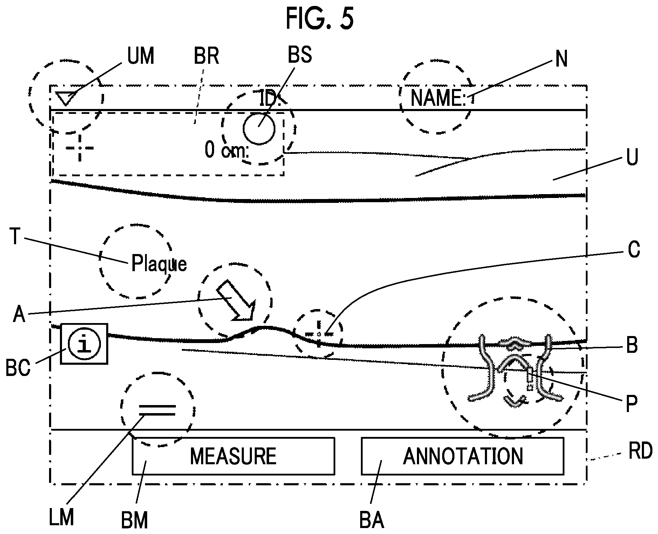

For example, as illustrated in Fig. 5, in a case in which a plurality of movable operation targets, such as the first caliper C and the measurement result box BR, are displayed in the image display region RD so as to be superimposed on the ultrasound diagnostic image U, the operation target generation unit 37 of the main-body-side processor 42 can generate a switching button BC for switching the operation targets whose operation is received by the operation target receiving unit 38. For example, the generated switching button BC is displayed in the image display region RD through the display control unit 33 as illustrated in Fig. 5. Here, the switching button BC may be displayed in the outer region RE, which is not illustrated.

In the example illustrated in Fig. 5, nine operation targets surrounded by dashed lines are displayed in the image display region RD. That is, six operation targets of the first caliper C, the measurement result box BR, the text T, the arrow A, the body mark B, and the probe mark P are displayed in the image display region RD as the operation targets that can be moved by the input operation of the user through the operation unit 40. Three operation targets of an upper menu button UM, a subject name input field N, and a lower menu button LM are displayed in the image display region RD as fixed operation targets that are not capable of being moved by the input operation of the user through the operation unit 40. For example, the upper menu button UM can be set such that a list of the inspection modes used for ultrasound diagnosis is displayed and not displayed in a case in which the upper menu button UM is touched by the user. For example, the subject name input field N can be set such that a user interface, such as a keyboard for inputting the name of the subject, is displayed in a case in which the subject name input field N is touched by the user. For example, the lower menu button LM can be set such that the measurement button BM and the annotation button BA are displayed and not displayed. Here, the dashed lines illustrated in Fig. 5 are illustrative. However, in practice, the dashed lines are not displayed in the image display region RD.

Whenever the switching button BC displayed in the image display region RD is touched by the user through the operation unit 40, the operation targets whose operation is received by the operation target receiving unit 38 are sequentially switched. For example, as illustrated in Fig. 5, a predetermined order is given to all of the nine operation targets displayed in the image display region RD. Therefore, whenever the switching button BC is touched by the user, the operation targets whose operation is received by the operation target receiving unit 38 can be sequentially switched according to the predetermined order.

Further, for example, a predetermined order is given to only six movable operation targets of the first caliper C, the measurement result box BR, the text T, the arrow A, the body mark B, and the probe mark P illustrated in Fig. 5, among the plurality of operation targets displayed in the image display region RD. Whenever the switching button BC is touched by the user, the operation target receiving unit 38 may sequentially switch the operation targets whose operation is received according to the predetermined order among the plurality of movable operation targets. In this case, the operation targets whose position is fixed, such as the upper menu button UM, the subject name input field N, and the lower menu button LM, may be set so to be freely touched by the user, without being affected by the fixation of the operation by the operation target receiving unit 38.

As such, even in a case in which a plurality of operation targets are displayed in the image display region RD, it is possible to prevent an input operation that is not intended by the user and to perform only the input operation intended by the user.

As described above, according to the ultrasound diagnostic apparatus 1 of Embodiment 2, in a case in which a plurality of movable operation targets are displayed in the image display region RD of the display unit 34, the switching button BC for sequentially switching the operation targets whose operation is received by the operation target receiving unit 38 is displayed on the display unit 34. Whenever the user operates the switching button BC through the operation unit 40, the operation targets whose operation is received by the operation target receiving unit 38 are switched. Therefore, even in a case in which a plurality of movable operation targets are displayed in the image display region RD, it is possible to perform only the input operation intended by the user.

The display aspect of the operation targets whose operation is received by the operation target receiving unit 38 of the main-body-side processor 42 among the plurality of operation targets displayed in the image display region RD of the display unit 34 can be set to be different from the display aspect of the other operation targets. For example, the operation targets whose operation is received by the operation target receiving unit 38 can be displayed in the following aspects: the operation targets are blinked for a predetermined time: the operation targets are displayed in a color different from the display color of the other operation targets; and the operation targets are surrounded by frames having any shape. Therefore, the user can easily understand the operation targets whose operation is received by the operation target receiving unit 38.

Further, in a case in which the switching button BC displayed on the display unit 34 is touched by the user, a plurality of operation targets displayed in the image display region RD may be displayed so as to be highlighted. For example, the plurality of operation targets are surrounded by frames having any shape, which makes it possible for the user to easily understand the plurality of operation targets displayed in the image display region RD. In this case, for example, the frames of the operation targets whose operation is received by the operation target receiving unit 38 are displayed in a display aspect different from the display aspect of the frames of the other operation targets, which makes it possible for the user to easily understand the operation targets whose operation is received by the operation target receiving unit 38.

Furthermore, for example, the number of the plurality of operation targets displayed in the image display region RD may be displayed on the display unit 34. In this case, an order may be given to the plurality of operation targets and the number of the operation target whose operation is currently received by the operation target receiving unit 38 in the given order may be displayed. In this case, the user can more easily understand the plurality of operation targets displayed in the image display region RD.

Further, in Embodiment 2, whenever the switching button BC displayed on the display unit 34 is operated by the user, the operation targets whose operation is received by the operation target receiving unit 38 are sequentially switched. However, a method for switching the operation targets whose operation is received by the operation target receiving unit 38 is not limited thereto.

For example, as illustrated in Fig. 5, all of the operation targets displayed in the image display region RD of the display unit 34, that is, all of six operation targets of the first caliper C, the measurement result box BR, the text T, the arrow A, the body mark B, and the probe mark P that are movable operation targets and three operation targets of the upper menu button UM, the subject name input field N, and the lower menu button LM that are operation targets whose position is fixed may be displayed in a state in which they can be selected by the touch operation of the user. In this state, the operation target receiving unit 38 may receive only the target touched by the user. In this case, the operation target receiving unit 38 fixes the operation of only the movable operation targets, and the operation targets whose position is fixed can be set so as to be freely touched by the user without being affected by the fixation of the operation by the operation target receiving unit 38.

Here, for example, the following may be set as a trigger for displaying a plurality of operation targets in the image display region RD in a state in which the operation targets can be selected by the touch operation of the user: the switching button BC is continuously touched by the user for a predetermined time; an empty region of the image display region RD, that is, the ultrasound diagnostic image U is continuously touched by the user for a predetermined time; and an empty region of the outer region RE is continuously touched by the user for a predetermined time.

As such, a plurality of operation targets are displayed in the image display region RD in a state in which they can be selected by the touch operation of the user, and the user selects one of the plurality of operation targets to switch the operation targets whose operation is received by the operation target receiving unit 38.

Therefore, even in a case in which the number of the plurality of operation targets displayed in the image display region RD is large, it is possible to more easily switch the operation targets whose operation is received by the operation target receiving unit 38.

### Embodiment 3

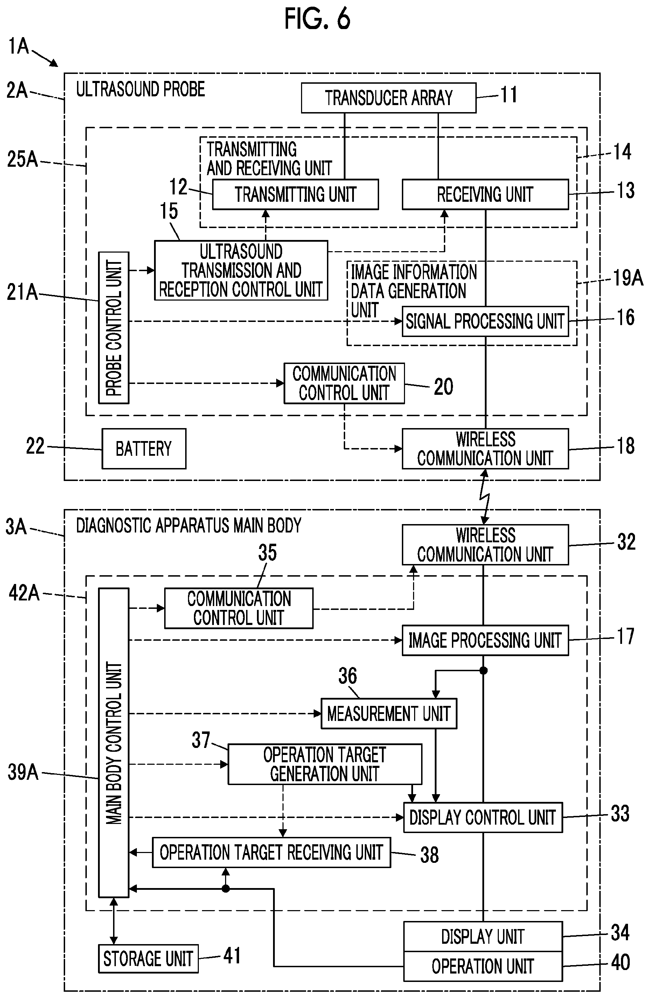

Fig. 6 illustrates the configuration of an ultrasound diagnostic apparatus 1A according to Embodiment 3. The ultrasound diagnostic apparatus 1A comprises an ultrasound probe 2A and a diagnostic apparatus main body 3A. The ultrasound probe 2A and the diagnostic apparatus main body 3A are connected by wireless communication.

As illustrated in Fig. 6, the ultrasound probe 2A according to Embodiment 3 is different from the ultrasound probe 2 according to Embodiment 1 illustrated in Fig. 1 in that the image processing unit 17 is removed and a probe control unit 21A is provided instead of the probe control unit 21.

In the ultrasound probe 2A, the wireless communication unit 18 is directly connected to the signal processing unit 16, and an image information data generation unit 19A is configured by the signal processing unit 16. A probe control unit 21A is connected to the transmitting unit 12, the receiving unit 13, the ultrasound transmission and reception control unit 15, the signal processing unit 16, and the communication control unit 20. Further, a probe-side processor 25A is configured by the transmitting and receiving unit 14, the ultrasound transmission and reception control unit 15, the signal processing unit 16, the communication control unit 20, and the probe control unit 21A.

The diagnostic apparatus main body 3A according to Embodiment 3 is different from the diagnostic apparatus main body 3 according to Embodiment 1 illustrated in Fig. 1 in that it comprises the image processing unit 17 between the wireless communication unit 32 and the display control unit 33 and comprises a main body control unit 39A instead of the main body control unit 39.

In the diagnostic apparatus main body 3A, the image processing unit 17 is connected to the wireless communication unit 32, and the display control unit 33 and the measurement unit 36 are connected to the image processing unit 17. Further, the main body control unit 39A is connected to the display control unit 33, the communication control unit 35, the measurement unit 36, the operation target generation unit 37, and the operation target receiving unit 38. Furthermore, a main-body-side processor 42A is configured by the display control unit 33, the communication control unit 35, the measurement unit 36, the operation target generation unit 37, the operation target receiving unit 38, and the main body control unit 39A.

The signal processing unit 16 of the image information data generation unit 19A corrects the attenuation of the sound ray signal generated by the beam former 28 of the receiving unit 13 caused by a propagation distance according to the depth of the position where the ultrasonic waves are reflected and performs an envelope detection process on sound ray signal to generate, as image information data, a signal that is tomographic image information related to the tissues in the subject.

The wireless communication unit 18 of the ultrasound probe 2A modulates a carrier on the basis of the signal generated by the signal processing unit 16 of the image information data generation unit 19A to generate a transmission signal indicating the image information data and wirelessly transmits the generated transmission signal to the wireless communication unit 32 of the diagnostic apparatus main body 3A.

The wireless communication unit 32 of the diagnostic apparatus main body 3A demodulates the transmission signal wirelessly transmitted from the wireless communication unit 18 of the ultrasound probe 2A to acquire the signal generated by the signal processing unit 16 of the image information data generation unit 19A and transmits the signal to the image processing unit 17 of the main-body-side processor 42A.

The image processing unit 17 of the main-body-side processor 42A raster-converts the signal transmitted from the wireless communication unit 32 of the diagnostic apparatus main body 3A into an image signal following the general television signal scanning method and performs various types of necessary image processing, such as brightness correction, gradation correction, sharpness correction, and color correction, on the generated image signal to generate an ultrasound image signal. Further, the image processing unit 17 transmits the generated ultrasound image signal to the display control unit 33 and the measurement unit 36.

The ultrasound image signal transmitted to the display control unit 33 is displayed as the ultrasound diagnostic image on the display unit 34 of the diagnostic apparatus main body 3A under the control of the display control unit 33.

The operation target generation unit 37 of the main-body-side processor 42A generates the operation targets displayed on the display unit 34 according to an input operation of the user through the diagnostic apparatus main body 3A. For example, as illustrated in Fig. 4, in a case in which the distance on the ultrasound diagnostic image U is measured by the touch of the measurement button BM by the user through the operation unit 40, the operation target generation unit 37 generates, as the operation targets, the first caliper C for measuring the distance between two points and the second caliper (not illustrated). In this way, the operation targets generated by the operation target generation unit 37 are displayed on the display unit 34 through the display control unit 33.

In a case in which the operation target generation unit 37 generates the operation target that is displayed so as to be superimposed on the ultrasound diagnostic image U displayed in the image display region RD of the display unit 34 and is movable, the operation target receiving unit 38 of the main-body-side processor 42A receives an operation performed on any position in the image display region RD by the user through the operation unit 40 as an operation of selecting the movable operation target. For example, as illustrated in Fig. 4, in a case in which the first caliper C is displayed as the movable operation target so as to be superimposed on the ultrasound diagnostic image U in the image display region RD, the operation target receiving unit 38 receives an operation performed on any position in the image display region RD by the user through the operation unit 40 as the operation of, for example, selecting and moving the first caliper C.

The measurement unit 36 of the main-body-side processor 42A measures, for example, the distance on the basis of the operation target which has been generated by the operation target generation unit 37 and operated by the user through the operation unit 40, such as a caliper for measuring the distance on the ultrasound diagnostic image, and transmits the measurement result to the display control unit 33. The measurement result transmitted to the display control unit 33 is displayed on the display unit 34 under the control of the display control unit 33.

As described above, even in the ultrasound diagnostic apparatus 1A according to Embodiment 3 in which the image processing unit 17 is not provided in the ultrasound probe 2A, but is provided in the diagnostic apparatus main body 3A, similar to the ultrasound diagnostic apparatus 1 according to Embodiment 1, in a case in which the operation target generation unit 37 generates the operation target that is superimposed on the ultrasound diagnostic image U displayed in the image display region RD of the display unit 34 and is movable, the operation target receiving unit 38 receives an operation performed on any position in the image display region RD by the user through the operation unit 40 as the operation of selecting and moving the movable operation target. Therefore, it is possible to prevent an input operation that is not intended by the user and to improve convenience in a case in which a touch operation is performed.

In the above-described Embodiments 1 and 2, the ultrasound image signal which has been subjected to the attenuation correction and the envelope detection process by the signal processing unit 16 of the image information data generation unit 19 and then subjected to raster conversion by the image processing unit 17 is wirelessly transmitted as the image information data from the wireless communication unit 18 of the ultrasound probe 2 to the diagnostic apparatus main body 3. In Embodiment 3, the signal subjected to the attenuation correction and the envelope detection process by the signal processing unit 16 of the image information data generation unit 19A is wirelessly transmitted as the image information data from the wireless communication unit 18 of the ultrasound probe 2A to the diagnostic apparatus main body 3A. However, it is preferable that the image information data wirelessly transmitted from the ultrasound probe 2 to the diagnostic apparatus main body 3 and the image information data wirelessly transmitted from the ultrasound probe 2A to the diagnostic apparatus main body 3A are signals after detection. However, the image information data is not limited to the signal after detection.

Further, in Embodiments 1 and 2, the ultrasound probe 2 and the diagnostic apparatus main body 3 are wirelessly connected to each other. In Embodiment 3, the ultrasound probe 2A and the diagnostic apparatus main body 3A are wirelessly connected to each other. However, the ultrasound probe 2 and the diagnostic apparatus main body 3 may be connected to each other in a wired manner, and the ultrasound probe 2A and the diagnostic apparatus main body 3A may be connected to each other in a wired manner. For example, each of the ultrasound probes 2 and 2A and the diagnostic apparatus main bodies 3 and 3A may be provided with a connection terminal to which a cable capable of transmitting information is connected. The ultrasound probe 2 and the diagnostic apparatus main body 3 may be connected to each other by the cable and the ultrasound probe 2A and the diagnostic apparatus main body 3A may be connected to each other by the cable.

In addition, the aspects of Embodiments 1 to 3 can be applied to portable ultrasound diagnostic apparatuses and can also be applied to stationary ultrasound diagnostic apparatuses.

### Explanation of References

1, 1A: ultrasound diagnostic apparatus
2, 2A: ultrasound probe
3, 3A: diagnostic apparatus main body
11: transducer array
12: transmitting unit
13: receiving unit
14: transmitting and receiving unit
15: ultrasound transmission and reception control unit
16: signal processing unit
17: image processing unit
18, 32: wireless communication unit
19, 19A: image information data generation unit
20, 35: communication control unit
21, 21A: probe control unit
22: battery
25, 25A: probe-side processor
26: amplification unit
27: AD conversion unit
28: beam former
33: display control unit
34: display unit
36: measurement unit
37: operation target generation unit
38: operation target receiving unit
39, 39A: main body control unit
40: operation unit
41: storage unit
42, 42A: main-body-side processor
B1: freeze button
B2: storage button
BA: annotation button
BC: switching button
BM: measurement button
BR: measurement result box
BS: measurement result movement button
C: caliper
LM: lower menu button
N: subject name input field
RD: image display region
RE: outer region
U: ultrasound diagnostic image
UM: upper menu button

## Claims

1. An ultrasound diagnostic apparatus (1, 1A) comprising:
a display unit (34) that has an image display region for displaying an acquired ultrasound diagnostic image;
an operation unit (40) that is disposed so as to be superimposed on the display unit (34) and is configured by a touch sensor used by a user to perform an input operation;
an operation target generation unit (37) that generates an operation target displayed on the display unit (34) according to an operation by the operation unit (40); and
an operation target receiving unit (38) that, in a case in which the operation target generation unit (37) generates an operation target that is superimposed on the ultrasound diagnostic image displayed in the image display region and is movable, prohibits an operation of operation targets other than the movable operation target and receives an operation performed on any position in the image display region through the operation unit (40) as an operation of selecting the movable operation target;
the ultrasound diagnostic apparatus being **characterized in that**, in a case in which a plurality of the movable operation targets are simultaneously displayed so as to be superimposed on the ultrasound diagnostic image:
the operation target receiving unit (38) sequentially fixes the operation of the movable operation targets according to a predetermined sequence: or
the operation target generation unit (37) generates a switching button for switching the operation targets which are displayed on the display unit (34) and whose operation is received by the operation target receiving unit (38), and the operation targets whose operation is received by the operation target receiving unit (38) are sequentially switched whenever the switching button is operated.

2. The ultrasound diagnostic apparatus (1, 1A) according to claim 1,
wherein the movable operation target is a region of interest for designating a partial region of the ultrasound diagnostic image in a case in which an inspection is performed in a brightness mode or a color Doppler mode.

3. The ultrasound diagnostic apparatus (1, 1A) according to claim 1,
wherein the movable operation target is a cursor for designating a position where a motion mode image is acquired in the ultrasound diagnostic image displayed in a brightness mode in a case in which an inspection is performed in a motion mode.

4. The ultrasound diagnostic apparatus (1, 1A) according to claim 1,
wherein the movable operation target is a cursor and a gate for designating a position where a pulse Doppler mode image is acquired in the ultrasound diagnostic image displayed in a brightness mode in a case in which an inspection is performed in a pulse Doppler mode.

5. The ultrasound diagnostic apparatus (1, 1A) according to claim 1,
wherein the movable operation target is an annotation.

6. The ultrasound diagnostic apparatus (1, 1A) according to claim 1,
wherein the movable operation target is a body mark and a probe mark that is displayed on the body mark.

7. The ultrasound diagnostic apparatus (1, 1A) according to claim 1,
wherein the movable operation target is a caliper for measurement.

8. The ultrasound diagnostic apparatus (1, 1A) according to any one of claims 1 to 7, further comprising:
an ultrasound probe and a diagnostic apparatus main body that are wirelessly connected to each other,
wherein the ultrasound probe includes:
a transducer array;
a transmitting and receiving unit (14) that transmits ultrasonic waves from the transducer array and generates a sound ray signal on the basis of a reception signal acquired by the transducer array;
an image information data generation unit (19, 19A) that generates image information data on the basis of the sound ray signal generated by the transmitting and receiving unit (14); and
a wireless communication unit that wirelessly transmits the image information data generated by the image information data generation unit (19, 19A) to the diagnostic apparatus main body,
the diagnostic apparatus main body includes:
the display unit (34) that displays an ultrasound image on the basis of the image information data wirelessly transmitted from the ultrasound probe;
the operation unit (40);
the operation target generation unit (37); and
the operation target receiving unit (38).

9. The ultrasound diagnostic apparatus (1, 1A) according to claim 8,
wherein the image information data is a signal obtained by performing attenuation correction according to a depth of a reflection position of the ultrasonic waves and an envelope detection process on the sound ray signal generated by the transmitting and receiving unit (14).

10. The ultrasound diagnostic apparatus (1, 1A) according to claim 8,
wherein the image information data is an ultrasound image signal obtained by performing attenuation correction according to a depth of a reflection position of the ultrasonic waves and an envelope detection process on the sound ray signal generated by the transmitting and receiving unit (14) and converting the sound ray signal according to a predetermined image display method.

11. A method for controlling an ultrasound diagnostic apparatus (1, 1A), the method comprising:
displaying an acquired ultrasound diagnostic image on a display unit (34);
generating an operation target displayed on the display unit (34) according to an operation by an operation unit (40) that is disposed so as to be superimposed on the display unit (34) and is configured by a touch sensor used by a user to perform an input operation; and
in a case in which an operation target that is superimposed on the ultrasound diagnostic image displayed in an image display region of the display unit (34) and is movable is generated, prohibiting an operation of operation targets other than the movable operation target and receiving an operation performed on any position in the image display region through the operation unit (40) as an operation of selecting the movable operation target;
the method being **characterized in that**, in a case in which a plurality of the movable operation targets are simultaneously displayed so as to be superimposed on the ultrasound diagnostic image, the method comprises:
sequentially fixing the operation of the movable operation targets according to a predetermined sequence: or
generating a switching button for switching the operation targets which are displayed on the display unit (34), and sequentially switching the operation targets whenever the switching button is operated.

## Patentansprüche

1. Ultraschalldiagnosevorrichtung (1, 1A), umfassend:
eine Anzeigeeinheit (34), die einen Bildanzeigebereich zum Anzeigen eines erfassten Ultraschalldiagnosebildes aufweist;
eine Betätigungseinheit (40), die so angeordnet ist, dass sie der Anzeigeeinheit (34) überlagert ist, und durch einen Berührungssensor konfiguriert ist, der von einem Benutzer verwendet wird, um eine Eingabebetätigung durchzuführen;
eine Betätigungsziel-Erzeugungseinheit (37), die ein Betätigungsziel, das auf der Anzeigeeinheit (34) angezeigt wird, gemäß einer Betätigung durch die Betätigungseinheit (40) erzeugt; und
eine Betätigungsziel-Empfangseinheit (38), die in einem Fall, in dem die Betätigungsziel-Erzeugungseinheit (37) ein Betätigungsziel erzeugt, das dem in dem Bildanzeigebereich angezeigten Ultraschalldiagnosebild überlagert ist und beweglich ist, eine Betätigung von Betätigungszielen, die von dem beweglichen Betätigungsziel verschieden sind, verbietet und eine Betätigung, die an beliebiger Position in dem Bildanzeigebereich durch die Betätigungseinheit (40) durchgeführt wird, als eine Betätigung des Auswählens des beweglichen Betätigungsziels empfängt;
wobei die Ultraschalldiagnosevorrichtung **dadurch gekennzeichnet ist, dass**:
in einem Fall, in dem mehrere der beweglichen Betätigungsziele gleichzeitig so angezeigt werden, dass sie dem Ultraschalldiagnosebild überlagert sind:
die Betätigungsziel-Empfangseinheit (38) die Betätigung der beweglichen Betätigungsziele gemäß einer vorbestimmten Sequenz sequentiell fixiert: oder
die Betätigungsziel-Erzeugungseinheit (37) eine Umschalttaste zum Umschalten der Betätigungsziele, die auf der Anzeigeeinheit (34) angezeigt werden und deren Betätigung von der Betätigungsziel-Empfangseinheit (38) empfangen wird, erzeugt und die Betätigungsziele, deren Betätigung von der Betätigungsziel-Empfangseinheit (38) empfangen wird, immer dann sequentiell umgeschaltet werden, wenn die Umschalttaste betätigt wird.

2. Ultraschalldiagnosevorrichtung (1, 1A) nach Anspruch 1,
wobei das bewegliche Betätigungsziel ein Bereich von Interesse zum Designieren eines Teilbereichs des Ultraschalldiagnosebildes in einem Fall, in dem eine Inspektion in einem Helligkeitsmodus oder einem Farb-Doppler-Modus durchgeführt wird, ist.

3. Ultraschalldiagnosevorrichtung (1, 1A) nach Anspruch 1,
wobei das bewegliche Betätigungsziel ein Cursor zum Designieren einer Position ist, an der ein Bewegungsmodus-Bild in dem Ultraschalldiagnosebild, das in einem Helligkeitsmodus angezeigt wird, erfasst wird, in einem Fall, in dem eine Inspektion in einem Bewegungsmodus durchgeführt wird.

4. Ultraschalldiagnosevorrichtung (1, 1A) nach Anspruch 1,
wobei das bewegliche Betätigungsziel ein Cursor und ein Gatter zum Designieren einer Position ist, an der ein Puls-Doppler-Modus-Bild in dem Ultraschalldiagnosebild, das in einem Helligkeitsmodus angezeigt wird, erfasst wird, in einem Fall, in dem eine Inspektion in einem Puls-Doppler-Modus durchgeführt wird.

5. Ultraschalldiagnosevorrichtung (1, 1A) nach Anspruch 1,
wobei das bewegliche Betätigungsziel eine Beschriftung ist.

6. Ultraschalldiagnosevorrichtung (1, 1A) nach Anspruch 1,
wobei das bewegliche Betätigungsziel eine Körpermarkierung und eine Sondenmarkierung ist, die auf der Körpermarkierung angezeigt wird.

7. Ultraschalldiagnosevorrichtung (1, 1A) nach Anspruch 1,
wobei das bewegliche Betätigungsziel ein Messschieber zum Messen ist.

8. Ultraschalldiagnosevorrichtung (1, 1A) nach einem der Ansprüche 1 bis 7, ferner umfassend:
eine Ultraschallsonde und einen Diagnosevorrichtungs-Hauptkörper, die miteinander drahtlos verbunden sind,
wobei die Ultraschallsonde enthält:
eine Wandleranordnung;
eine Übertragungs- und Empfangseinheit (14), die Ultraschallwellen von der Wandleranordnung überträgt und ein Schallstrahlensignal auf der Grundlage eines Empfangssignals, das von der Wandleranordnung erfasst wird, erzeugt;
eine Bildinformationsdaten-Erzeugungseinheit (19, 19A), die Bildinformationsdaten auf der Grundlage des von der Übertragungs- und Empfangseinheit (14) erzeugten Schallstrahlensignals erzeugt; und
eine drahtlose Kommunikationseinheit, die die von der Bildinformationsdaten-Erzeugungseinheit (19, 19A) erzeugten Bildinformationsdaten drahtlos an den Diagnosevorrichtungs-Hauptkörper überträgt,
wobei der Diagnosevorrichtungs-Hauptkörper enthält:
die Anzeigeeinheit (34), die ein Ultraschallbild auf der Grundlage der Bildinformationsdaten, die von der Ultraschallsonde drahtlos übertragen werden, anzeigt;
die Betätigungseinheit (40);
die Betätigungsziel-Erzeugungseinheit (37); und
die Betätigungsziel-Empfangseinheit (38).

9. Ultraschalldiagnosevorrichtung (1, 1A) nach Anspruch 8,
wobei die Bildinformationsdaten ein Signal sind, das durch Durchführen von Dämpfungskorrektur gemäß einer Tiefe einer Reflexionsposition der Ultraschallwellen und eines Hüllkurven-Detektionsprozesses an dem Schallstrahlensignal, das durch die Übertragungseinheit (14) erzeugt wird, erhalten wird.

10. Ultraschalldiagnosevorrichtung (1, 1A) nach Anspruch 8,
wobei die Bildinformationsdaten ein Ultraschallbildsignal sind, das durch Durchführen von Dämpfungskorrektur gemäß einer Tiefe einer Reflexionsposition der Ultraschallwellen und eines Hüllkurven-Detektionsprozesses an dem Schallstrahlensignal, das durch die Übertragungs- und Empfangseinheit (14) erzeugt wird, und Umwandeln des Schallstrahlensignals gemäß eines vorbestimmten Bildanzeigeverfahren erhalten wird.

11. Verfahren zum Steuern einer Ultraschalldiagnosevorrichtung (1, 1A), wobei das Verfahren umfasst:
Anzeigen eines erfassten Ultraschalldiagnosebildes auf einer Anzeigeeinheit (34);
Erzeugen eines Betätigungsziels, das auf der Anzeigeeinheit (34) angezeigt wird, gemäß einer Betätigung durch eine Betätigungseinheit (40), die so angeordnet ist, dass sie der Anzeigeeinheit (34) überlagert ist, und durch einen Berührungssensor konfiguriert ist, der von einem Benutzer verwendet wird, um eine Eingabebetätigung durchzuführen; und
in einem Fall, in dem ein Betätigungsziel, das dem Ultraschalldiagnosebild, das in einem Bildanzeigebereich der Anzeigeeinheit (34) angezeigt wird, überlagert ist und beweglich ist, erzeugt wird, Verbieten einer Betätigung von Betätigungszielen, die von dem beweglichen Betätigungsziel verschieden sind, und Empfangen einer Betätigung, die an beliebiger Position in dem Bildanzeigebereich durch die Betätigungseinheit (40) durchgeführt wird, als eine Betätigung des Auswählens des beweglichen Betätigungsziels;
wobei das Verfahren **dadurch gekennzeichnet ist, dass**:
in einem Fall, in dem mehrere der beweglichen Betätigungsziele gleichzeitig so angezeigt werden, dass sie dem Ultraschalldiagnosebild überlagert sind, das Verfahren umfasst:
sequentielles Fixieren der Betätigung der beweglichen Betätigungsziele gemäß einer vorbestimmten Sequenz: oder
Erzeugen einer Umschalttaste zum Umschalten der Betätigungsziele, die auf der Anzeigeeinheit (34) angezeigt werden, und sequentielles Umschalten der Betätigungsziele, immer dann, wenn die Umschalttaste betätigt wird.

## Revendications

1. Appareil de diagnostic par ultrasons (1, 1A) comprenant :
une unité d'affichage (34) qui comporte une région d'affichage d'image pour afficher une image de diagnostic par ultrasons acquise ;
une unité d'opération (40) qui est disposée de manière à être superposée sur l'unité d'affichage (34) et est configurée par un capteur tactile utilisé par un utilisateur pour effectuer une opération d'entrée ;
une unité de génération de cible d'opération (37) qui génère une cible d'opération affichée sur l'unité d'affichage (34) en fonction d'une opération par l'unité d'opération (40) ; et
une unité de réception de cible d'opération (38) qui, dans un cas où l'unité de génération de cible d'opération (37) génère une cible d'opération qui est superposée sur l'image de diagnostic par ultrasons affichée dans la région d'affichage d'image et
est mobile, interdit une opération de cibles d'opération autres que la cible d'opération mobile et reçoit une opération effectuée sur n'importe quelle position dans la région d'affichage d'image par l'unité d'opération (40) comme une opération de sélection de la cible d'opération mobile ;
l'appareil de diagnostic par ultrasons étant **caractérisé en ce que**,
dans un cas où une pluralité des cibles de opération mobiles sont affichées simultanément de manière à être superposées sur l'image de diagnostic par ultrasons :
l'unité de réception de cible d'opération (38) fixe séquentiellement l'opération des cibles d'opération mobiles selon une séquence prédéterminée : ou
l'unité de génération de cible d'opération (37) génère un bouton de commutation pour commuter les cibles d'opération qui sont affichées sur l'unité d'affichage (34) et dont l'opération est reçue par l'unité de réception de cible d'opération (38), et les cibles d'opération dont l'opération est reçue par l'unité de réception de cible d'opération (38) sont commutées séquentiellement chaque fois que le bouton de commutation est actionné.

2. Appareil de diagnostic par ultrasons (1, 1A) selon la revendication 1,
dans lequel la cible d'opération mobile est une région d'intérêt pour désigner une région partielle de l'image de diagnostic par ultrasons dans un cas où une inspection est effectuée dans un mode de luminosité ou un mode Doppler couleur.

3. Appareil de diagnostic par ultrasons (1, 1A) selon la revendication 1,
dans lequel la cible d'opération mobile est un curseur pour désigner une position où une image de mode de mouvement est acquise dans l'image de diagnostic par ultrasons affichée dans un mode de luminosité dans un cas où une inspection est effectuée dans un mode de mouvement.

4. Appareil de diagnostic par ultrasons (1, 1A) selon la revendication 1,
dans lequel la cible d'opération mobile est un curseur et une porte pour désigner une position où une image de mode Doppler pulsé est acquise dans l'image de diagnostic par ultrasons affichée dans un mode de luminosité dans un cas où une inspection est effectuée dans un mode Doppler pulsé.

5. Appareil de diagnostic par ultrasons (1, 1A) selon la revendication 1,
dans lequel la cible d'opération mobile est une annotation.

6. Appareil de diagnostic par ultrasons (1, 1A) selon la revendication 1,
dans lequel la cible d'opération mobile est une marque de corps et une marque de sonde qui est affichée sur la marque de corps.

7. Appareil de diagnostic par ultrasons (1, 1A) selon la revendication 1,
dans lequel la cible d'opération mobile est un pied à coulisse pour la mesure.

8. Appareil de diagnostic par ultrasons (1, 1A) selon l'une quelconque des revendications 1 à 7, comprenant en outre :
une sonde ultrasonore et un corps principal d'appareil de diagnostic qui sont connectés sans fil l'un à l'autre,
dans lequel la sonde ultrasonore inclut :
un réseau de transducteurs ;
une unité de transmission et de réception (14) qui transmet des ondes ultrasonores depuis le réseau de transducteurs et génère un signal de rayon sonore sur la base d'un signal de réception acquis par le réseau de transducteurs ;
une unité de génération de données d'informations d'image (19, 19A) qui génère des données d'informations d'image sur la base du signal de rayon sonore généré par l'unité de transmission et de réception (14) ; et
une unité de communication sans fil qui transmet sans fil les données d'informations d'image générées par l'unité de génération de données d'informations d'image (19, 19A) au corps principal d'appareil de diagnostic,
dans lequel le corps principal d'appareil de diagnostic inclut :
l'unité d'affichage (34) qui affiche une image ultrasonore sur la base des données d'informations d'image transmises sans fil depuis la sonde ultrasonore ;
l'unité d'opération (40) ;
l'unité de génération de cible d'opération (37) ; et
l'unité de réception de cible d'opération (38).

9. Appareil de diagnostic par ultrasons (1, 1A) selon la revendication 8,
dans lequel les données d'informations d'image sont un signal obtenu en effectuant une correction d'atténuation en fonction d'une profondeur d'une position de réflexion des ondes ultrasonores et un processus de détection d'enveloppe sur le signal de rayon sonore généré par l'unité de transmission et de réception (14).

10. Appareil de diagnostic par ultrasons (1, 1A) selon la revendication 8,
dans lequel les données d'informations d'image sont un signal d'image ultrasonore obtenu en effectuant une correction d'atténuation en fonction d'une profondeur d'une position de réflexion des ondes ultrasonores et un processus de détection d'enveloppe sur le signal de rayon sonore généré par l'unité de transmission et de réception (14) et en convertissant le signal de rayon sonore selon un procédé d'affichage d'image prédéterminé.

11. Procédé de contrôle d'un appareil de diagnostic par ultrasons (1, 1A), le procédé comprenant :
afficher une image de diagnostic par ultrasons acquise sur une unité d'affichage (34) ;
générer une cible d'opération affichée sur l'unité d'affichage (34) en fonction d'une opération par une unité d'opération (40) qui est disposée de manière à être superposée sur l'unité d'affichage (34) et est configurée par un capteur tactile utilisé par un utilisateur pour effectuer une opération d'entrée ; et
dans un cas où une cible d'opération qui est superposée sur l'image de diagnostic par ultrasons affichée dans une région d'affichage d'image de l'unité d'affichage (34) et est mobile est générée, interdire une opération de cibles d'opération autres que la cible d'opération mobile et recevoir une opération effectuée sur n'importe quelle position dans la région d'affichage d'image par l'unité d'opération (40) comme une opération de sélection de la cible d'opération mobile ;
le procédé étant **caractérisé en ce que**,
dans un cas où une pluralité des cibles de opération mobiles sont affichées simultanément de manière à être superposées sur l'image de diagnostic par ultrasons,
le procédé comprend :
fixer séquentiellement l'opération des cibles d'opération mobiles selon une séquence prédéterminée : ou
générer un bouton de commutation pour commuter les cibles d'opération qui sont affichées sur l'unité d'affichage (34), et commuter séquentiellement les cibles d'opération chaque fois que le bouton de commutation est actionné.
